# EUROPEAN PATENT SPECIFICATION

(11) **EP 0 238 553 B1**
(45) Date of publication and mention of the grant of the patent: **07.05.1997**
(21) Application number: 86905644.0
(22) Date of filing: 12.09.1986
(51) Int. Cl.: A61K 31/16, A61K 31/195, A61M 5/14, A61M 37/00, A61M 3/00

(54) **METHOD OF TREATING CATABOLIC DYSFUNCTION**
BEHANDLUNGSVERFAHREN KATABOLER DYSFUNKTION
TRAITEMENT D'UN DEREGLEMENT CATABOLIQUE

(30) Priority: 12.09.1985 US 775214
(43) Date of publication of application: 30.09.1987
(73) Proprietor: BRIGHAM AND WOMEN'S HOSPITAL, Boston, Massachusetts 02115 (US)
(72) Inventor: WILMORE, Douglas, W., Brookline, MA 02146 (US); SMITH, Robert, J., Brookline, MA 02146 (US)
(74) Representative: Wibbelmann, Jobst, Dr., Dipl.-Chem.
(86) International application number: US8601870
(87) International publication number: WO8701589

(56) References cited:
- EP-A- 0 059 694
- US-A- 1 909 591
- US-A- 2 868 693
- US-A- 3 195 778
- US-A- 3 217 711
- US-A- 3 982 534
- US-A- 4 200 095
- US-A- 4 265 240
- US-A- 4 334 535
- US-A- 4 396 383
- HOPPE-SEYLER'S Z. PHYSIOL. CHEM., vol. 364, 1983, pages 1253-1254; I. AMBERGER et al.: "The potential patenteral application of the peptide L-alanyl-L- glutamine as a nitrogen source in severe catabolic states"
- JOURNAL OF PARENTERAL AND ENTERAL NUTRITION, vol. 9, no. 5, September/October 1985, pages 583-589; R. KAPADIA et al.: "Maintenance of skeletal muscle intracellular glutamine during standard surgical trauma"
- JAP. J. PHARMACOLOGY, vol. 24, no. 1, 1974, pages 169-171; S. OKABE et al.: "Effect of L-glutamine on indomethacin-induced gastric lesions in the rat"
- AKT. ERNAHR., vol. 9, 1984, pages 147-149, Georg Thieme Verlag, Stuttgart, DE; S. ALBERS et al.: "Komplette parenterale Ernährung mit und ohne einem synthetischen Dipeptid(L-alanyl-L-glutamin) bei Ratten mit experimentaller Katabolie"
- NUTRITION REPORTS INTERNATIONAL, vol. 23, no. 4, 1981, pages 749-753; R.F. DERR et al.: "Total parenteral nutrition: Improved formula for healing rat jejunal anastomoses"
- CHEMICAL ABSTRACTS, Volume 104, No. 25, issued 23 June 1986, Columbus, Ohio, US; MOYER et al.: "Effects of Gastrin, Glutamine, and Somatostastin on the in Vitro Growth of Normal and Malignant Human Gastric Mucosal Cells", see page 106, colmn 2, No. 219523X, Arch. Surg., 1986, 121(3), pages 285-8
- CHEMICAL ABSTRACTS, Volume 104, No. 15, issued 14 April 1986, Columbus, Ohio, US; VIALLARD et al.: "Effect of Glutamine Deprivation and Glutamate or Ammonium Chloride Addition on Growth Rate, Metabolism and Differentiation of Human Colon Cancer Cell-Line H T29", see page 492, column 2, No. 127504p
- Biological Abstracts, Volume 71, No. 6, issued 15 March 1981; CLOWES et al.: "Effects of patenteral alimentation on amino acid metabolism in septic patients", column 1, no. 39209
- CHEMICAL ABSTRACTS, Volume 103, NO. 6, issued 05 August 1985; OHTA et al.: "Effect of L-Glutamate, Injected into the Posterior Hypothalamus, on Heart Rate in Unanesthetized and Unrestrained Rats", see column 2, page 81, No. 32689t
- Biological Abstratcs, Volume 71, No. 3, issued 01 February 1981; TISCHLEN et al.: "Leucine Degradation and Release of Glutamine and Alanine by Adipose Tissue", column 2, No. 14875
- Biological Abstracts, volume 79, No. 7, issued 01 April 1985; SCHWARTAU et al.: "An Experimental Animal Study of Chronic Lack of Thiamin: Alterations in Carbohydrate and Amino-Acid Metabolism at and under Strain", column 2, No. 61030
- Biological Abstracts, Volume 72, No. 4, issued 01 October 1981; DERR et al.: "TPN: Improved Formula for Heading Rat jejunal anastomosis", column 1, No. 47176
- Biological Abstracts, Volume 78, No. 9, issued 01 October 1984; HONG et al.: "Metabolic effects of exhaustive training of athletes", column 2, no. 70581
- Biological Abstract 71:39209; CLOWES et al.: "Effects of Parenteral Alimentation on Amino Acid Metabolism in Septic Patients", Surgery (1980), Volume 88, No. 4
- Biological Abstracts 57:21213; SCHREK et al.: "Effect of Asparagine and Glutamine Deficiency on Normal and Leukemic Cells", J. Natl Cancer Inst, 51(4) 1973
- Biological Reviews, BR31:29511; CRAWFORD et al.: "The Effect of L-Glutamine Deficiency and Antagonists on Chronic Lymphocytic Leukemia Cells in Vitro", Amer. Assioc for Cancer Research Annual Meet., 1986
- Biological Reviews, BR 31:47304; SMITH et al.: "Tissue Specific Nutritional Regulation of Glutamine Metabolism", Amer. Fed Clinical Research, Clin Res Volume 34, No. 2, 1986; Same as BR 31:65644, Am. J. Clin Nat. 43(4), 1986
- Biological Abstracts, Volume 70, No. 8, issued 15 October 1980; ZANELLO et al.: "Alterations in the enzyme profile in intensive care patients undergoing TPN", column 2, no. 52747
- Biological Abstracts, Volume 72, No. 4, issued 15 August 1981, ROTH et al, "Biochemical Methods for the Determination of a Clinical Protein Catabolism", column 1, No. 25963
- Biological Abstracts, Volume 79, No. 9, issued 01 May 1985; MILAKOFSKY et al.: "Rat Plasma Labels of Amino Acids and Related Compounds during Stress", column 1, No: 79680
- Biological Abstracts, Volume 80, No. 11, issued 01 December 1985; ROTH et al.: "Metabolic Responses to Severe Infection and Sepsis", column 1, No. 96352
- Biological Abstracts, Volume 80, No. 4, issued 15 August 1985; IVANOVA et al.: "Comparative Study of some Drugs on Different Models of Brain Hypoxia", column 2, No: 34601
- Biological Abstracts, Volume 79, No. 10, issued 15 May 1985; BERGNER et al.: "Influence of the glutamic acid content of the diet on the catabolic rate of labeled glutamic acid in rats", column 1, no. 88557
- Biological Review, 27:72936; MILAKOFSKY et al.: "Amino-Acids and Related Compounds on Rat Plasma during Stress", 1984, Fed. Proc. 43(3)
- Biological Abstracts, Volume 80, No. 11, issued 01 December 1985; ROTH et al.: "Changes of the Protein Metabolism in Cachexia and Catabolism", column 1, No. 96470
- Biological Abstracts 63:17129; OKABE et al.: "Effects on Various Gastric Lesions in Rats and Quinea Pigs", Digestion 14(4), 1976 pages 325-331
- Biological Reviews, BR 11:24358; HARADA et al.: "Changes in Biological Elements in Mouse Stress Ulcer and Effects of Drugs", Folca Pharmacol JPN 69(6), 1973
- Biological Reviews, BR 10:24019; LONGTON et al.: "Initation of Gingival Lesions during Amino-Acid Stress", J Dent Res 52, 1973
- Biological Abstracts, BA 53:61561; HEATH et al.: "The Effects of the Stress Caused by Experimental Procedures on Alanine, Aspartate Glutanate and Glutamine in Rat Liver", Biochem. J. 125 (3), pages 765-771, 1971
- CHEMICAL ABSTRACTS, volume 102, no. 3, issued 21 January 1985, Columbus, Ohio, US; ALBERS et al.: "Complete Parenteral Feeding with and without a Synthetic Dipeptide in Rats with Experimental Catabolism", see page 583, column 2, Abstract No. 23355h
- CHEMICAL ABSTRACTS, volume 103, no. 13, issued 30 September 1985, Columbus, Ohio, US; ELIA et al.: "Alanine and Glutamine Release from the Human Forearm: Effects of Glucose Administration", see page 423, column 1 No. 102688t

## Description

The present invention relates to the use of glutamine or a functional analogue thereof that retains the characteristics of glutamine in the preparation of an agent for treating or preventing tissue damage in an animal afflicted with an atrophy caused by catabolic dysfunction.

It is known from the state of the art that certain catabolic dysfunctions following surgery, peptic ulcers and gastric lesions may be alleviated by administrating glutamine containing compositions; as described by the following documents:
- NUTRITION REPORTS INTERNATIONAL, vol. 23, n°4, 1981, p. 749-753,
- J. OF PARENTERAL AND ENTERAL NUTRITION, vol. 9, n°5, 1985, p. 583-589,
- US-A- 2 868 693,
- AKT. ERNÄHR., vol. 9, 1984, p. 147-149,
- HOPPE - SEYLER'S Z. PHYSIOL. CHEM., vol. 364, 1983, p. 1253,
- JAP. J. PHARMACOLOGY, vol. 24, n°1, 1974, p. 169-171.
However, none of these prior art documents disclose that an atrophy which may occur following catabolic dysfunction can be treated by administering glutamine or glutamine analogues.

Glutamine is a non-essential amino acid which can be synthesized by most tissues. Unlike most amino acids, glutamine has two amine moieties: an alpha-amino group and an amide group. It is the presence of the amide group which enables glutamine to remove ammonia from the peripheral tissues of the body and transport nitrogen to visceral organs. In addition, it is common for tissues that remove glutamine from the circulation to utilize the carbon skeleton for energy.

Glutaminase and glutamine synthetase are the two principal enzymes involved in the regulation of glutamine metabolism. Glutaminase catalyzes the hydrolysis of glutamine to glutamate and ammonia, while glutamine synthetase catalyzes the synthesis of glutamine from glutamate and ammonia. While most tissues have both of these enzymes, usually one is more active than the other, depending on the particular tissue.

Glutamine synthesis and exportation occurs primarily in skeletal muscle and the brain. In turn, glutamine is consumed by such replicating cells as fibroblasts, lymphocytes, tumor cells and intestinal epithelial cells. Characteristically, these cells possess high levels of glutaminase activity and low levels of intracellular glutamine. This fact may also be clinically significant for patients having large wounds, inflammation associated with infection, or a gastrointestinal dysfunction which precludes normal enteral feeding since, in these cell types, the desirable proliferation of cells in these conditions may depend on the availability of sufficient levels of glutamine.

In the gastrointestinal tract, glutamine is used as a respiratory fuel. The enteral administration of glutamine results in increased uptake of luminal glutamine by the gut mucosa accompanied by a simultaneous decrease in uptake of glutamine from the circulation. Thus, the consumption of glutamine by the gut is balanced between these two sources of glutamine.

Most of the glutamine taken up by the gastrointestinal tract occurs via the epithelial cells lining the villi of the small intestine. The glutamine metabolism which occurs in the small intestine provides a major source of energy for the gut and produces precursors for hepatic ureagenesis and gluconeogenesis by processing nitrogen and carbon from other tissues.

Evidence of the essential role of glutamine in the maintenance of normal intestinal structure and function was suggested in a study by Baskerville et al., British Journal of Experimental Pathology, 61: 132 (1980). These authors lowered the concentration of plasma glutamine to undetectable levels by infusing purified glutaminase into rhesus monkeys, marmosets, rabbits, and mice. As a result of this treatment, these animals displayed vomiting, diarrhea, villus atrophy, mucosal ulcerations, and intestinal necrosis.

Martin et al. (U.S. Patent 2,283,817) disclose a composition containing glutamine which is used as a detoxicant, rather than a dietary supplement. In the patent, glutamine is combined synergistically with other amino acids to act directly on a toxin to inhibit any deleterious effect.

In Shive et al. (U.S. Patent 2,868,693), the patentees disclose glutamine-containing compositions for the treatment of peptic ulcers.

Further evidence of the potential protective effect of glutamine was shown by Okabe et al., Digestive Disease, 20: 66 (1975), who found that glutamine could protect against aspirin-induced gastric ulcerations in humans.

This visceral glutamine requirement may be even greater during critical illness, when glutamine metabolism by the small intestine is known to be increased (Souba et al., Surgery, 94(2): 342 (1983)).

At present, the nutritional requirements of patients who are unable to feed themselves adequately are met through the administration of enteral or parenteral diets. Enteral diets are usually administered using small-bore tubing which is placed through the nose into the gastric, or duodenal regions, or through surgical implantation as in, for example, gastrostomy, or jejunostomy. Those enteral formulas which are presently available can be divided into four basic categories: elemental, polymeric, modular, and altered amino acids. These formulae contain glutamine. The levels of nutrients present in the enteral diets, however, are generally based upon the dietary requirements of a normal individual and not that of a patient suffering from a catabolic disease.

Elemental formulas require minimal digestive action and are composed primarily of small peptides and/or amino acids, glucose oligosaccharides, and vegetable oil or medium-chain triglycerides.

In polymeric formulas, complex nutrients such as, for example, soy protein, lactalbumin, or casein are utilized as a source of protein; maltodextrins, or corn syrup solids as a source of carbohydrate; and vegetable oils, or milk fat as a source of fat.

Modular diets can be produced by combining protein, carbohydrate, or fat with a monomeric or polymeric formula to meet special nutritional requirements.

Formulas which are composed of altered amino acid compositions are used primarily for patients with genetic errors of nitrogen metabolism or acquired disorders of nitrogen accumulation, the object here being to limit the intake by the patient of certain amino acids which may be detrimental.

Parenteral diets are usually administered intravenously. These intravenous fluids are sterile solutions composed of simple chemicals such as, for example, sugars, amino acids, and electrolytes, which can be easily assimilated.

The term "total parenteral nutrition" (TPN) is used to describe formulas for use in patients who derive their entire dietary requirements intravenously. Total parenteral nutrition formulas, unlike enteral formulas, do not normally contain glutamine. The absence of glutamine from parenteral formulas is due, in part, to concern with respect to its instability at room temperature, and the resulting generation of ammonia and pyroglutamic acid. There has also been concern about the generation of glutamic acid from glutamine because of the potential toxicity of glutamic acid as a neurotransmitter. In fact, these concerns do not appear to be justified. At a pH just below neutrality, glutamine degrades very slowly (Souba, S.C.D. Thesis in Harvard Medical School Library, June, 1984).

Total parenteral nutrition results in villus atrophy, a phenomenon which is generally reversible when oral feedings are resumed. Since TPN formulas lack glutamine, the body's requirements for this amino acid must be derived from synthetic pathways in body tissues.

In patients with critical illnesses, net protein catabolism is associated with markedly diminished muscle glutamine pools (Askanazi et al., Annals of Surgery, 192: 78 (1980); Askanazi et al., Annals of Surgery, 191: 465 (1980)), reduced plasma levels of glutamine (Askanazi et al., Annals of Surgery, 192: 78 (1980); Askanazi et al., Annals of Surgery, 191: 465 (1980)), and a presumed increase in intestinal glutamine utilization (Souba et al., Archives of Surgery, 120: 66 (1985); Souba et al., Surgery, 94(2): 342 (1983)). Glucocorticoids are known to increase glutamine consumption by the small intestine (Souba et al., Surgical Forum, 34: 74 (1983)).

None of the prior art studies have shown that the atrophy of intestinal villi, which occur during total parenteral nutrition, can be prevented through the administration of high levels of glutamine.

### SUMMARY OF THE INVENTION

In the invention, an animal having, or at risk of having, an atrophy of the small intestinal mucosa being caused by a catabolic dysfunction is given a therapeutically effective amount of glutamine. This amount of glutamine is greater than that normally encountered in the diet of healthy individuals. This increased level of glutamine is necessary to compensate for the greater demand for glutamine which occurs during certain catabolic dysfunctions. In the absence of exogenous glutamine during these catabolic dysfunctions, glutamine would be derived through the breakdown of muscle tissue. The decline in glutamine concentrations in the plasma in spite of accelerated glutamine release from muscle during catabolic dysfunctions indicates systemic glutamine deficiency. In spite of accelerated glutamine release from muscle, intestinal mucosal cell demand exceeds the supply. This, in turn, predisposes to intestinal villus atrophy.

Thus, the present invention concerns the use of glutamine or a functional analogue thereof that retains the characteristics of glutamine in the preparation of an agent for treating atrophy of small intestinal mucosa, the atrophy caused by catabolic dysfunction.

Provision of exogenous glutamine to a stressed patient may better support the metabolic requirements of the small intestine and possibly decrease the rate of systemic protein catabolism.

### DESCRIPTION OF THE FIGURES

Figure 1 is a graphic presentation of the effect of oral diet on plasma glutamine levels after subtotal small intestine resection.

Figure 2 is a graphic presentation of data demonstrating the effect of oral diet on distal ilium weight after subtotal small intestine resection.

Figure 3 is a graphic presentation of data demonstrating the effect of oral diet on mucosal thickness of distal ilium after subtotal resection of small intestine.

Figure 4 is a graphic presentation of the effect of oral diet on villus height in distal ilium after subtotal resection of small intestine.

### BRIEF DESCRIPTION OF THE PREFERRED EMBODIMENTS

The inventors have devised a new use of glutamine in the treatment of atrophy of the mucosa of the small intestine caused by catabolic dysfunctions. The present invention comprises the administration to an animal afflicted with, or likely to develop, an atrophy of the intestinal mucosa, a therapeutically effective amount of glutamine or a functional analogue thereof. This therapeutically effective amount is greater than that present in a normal diet. The normal dietary intake for humans is about 2 to 4 g/day.

A catabolic dysfunction is a condition which induces a catabolic biochemical pathway in which the degradation of an anatomical structure occurs. The dietary administration of glutamine appears to satisfy the biochemical requirements of these catabolic conditions such that it is not necessary for the body to synthesize glutamine or to obtain glutamine from the breakdown of skeletal muscle.

The present invention is intended to be used in all catabolic dysfunctions where there is an increased demand for glutamine and an atrophy of the small intestinal mucosa occurs. These catabolic dysfunctions can be either enteral or parenteral. For example, the atrophy of the villi of the small intestine which occurs when TPN diets are administered is an enteral catabolic dysfunction. Atrophy of the villi does not usually occur due to direct catabolic activity on the enterocytes, but rather, is due to the lack of glutamine in the diet of patients on a parenteral diet.

Parenteral catabolic dysfunctions which display increased demand for glutamine occur during or following surgery, sepsis, burn injuries, anorexia, and uncontrolled diabetes.

Animals in which the invention is effective are those commonly classified as mammals, including humans.

The term "enteral" is intended to indicate that portion of the alimentary canal between the stomach and the anus.

The term "parenteral" denotes that region outside of the digestive tract.

The administration of glutamine can be by both enteral and parenteral means.

An example of how the enteral administration of glutamine is accomplished is by using small-bore tubing placed via the nose into the gastric or duodenal regions, or through surgical implantation as in, for example, gastrostomy, or jejunostomy.

Examples of parenteral routes of administration include, but are not limited to, such routes as subcutaneous, intramuscular, or intravenous injection, nasopharyngeal or mucosal absorption, or transdermal absorption. In most cases, the glutamine is administered intravenously. In intravenous administration, the therapeutically effective amount of glutamine is in a liquid form which is administered from a reservoir directly via the placement of a needle into a large vein of the patient, wherein the needle is connected to the reservoir by tubing.

Regardless of which route of administration is utilized, the glutamine can be administered either singly or as a dietary supplement. When used as a dietary supplement, the glutamine can be mixed with an existing enteral or parenteral diet prior to administration to the patient. It is also possible to administer the glutamine without mixing it directly with the other components of a diet as, for example, in intravenous feeding wherein the glutamine is not directly added to he main intravenous bottle, but instead is added to a common reservoir using a "piggy-back" bottle.

Functional analogues, derivatives, substitution products, isomers, or homologues of glutamine which retain the characteristics of glutamine are contemplated as equivalents. Preferred are those analogues capable of donating an amine group and being metabolized in the Krebs cycle. Most preferred are compounds which possess the amino acid residue at one terminus of a carbon chain and an amine moiety at the other terminus of the carbon chain.

The therapeutically effective dose ranges for the administration of glutamine are those large enough to prevent the atrophy of the small intestinal mucosa of the body in order to maintain metabolic homeostasis. In an enteral diet glutamine would be administered at a rate greater than or equal to 0.3 grams per kilogram of body weight per day. Such administration rates could be 0.3 to 2.0 grams per kilogram of body weight per day, preferably 0.3 to 1.5 grams per kilogram of body weight per day, and more preferably 0.4 to 1.0 grams per kilogram of body weight per day. The rate of administration for glutamine when administered intravenously would be greater than or equal to 0.1 grams per kilogram of body weight per day. Such administration rates could be 0.2 to 3.0 grams per kilogram of body weight per day, preferably 0.3 to 2.5 grams per kilogram of body weight per day, more preferably 0.4 to 2.0 grams per kilogram of body weight per day.

According to the method of the invention, glutamine may be administered by simply modifying existing dietary formulas to contain the proper concentration of glutamine. Most preferably, the glutamine would remain in a dry form such as, for example, a sterile lyophilized powder which is aseptically hydrated at the time of administration and mixed at the proper concentration with the other components of the dietary composition. Alternatively, the glutamine could be premixed with the other components of a dry formula which is aseptically rehydrated at time of administration, or stored as a frozen concentrate which is thawed and mixed at the proper concentration at time of use.

The use of glutamine by the method according to the invention is ideally suited for the preparation of compositions. These compositions may comprise glutamine alone or in combination with other chemicals. These other chemicals can be pharmaceutically acceptable carriers, as well as other active substances of the diet as, for example, free amino acids, protein hydrolysates, or oils.

Preparations for parenteral administration include sterile aqueous or non-aqueous solutions, suspensions, and emulsions. Carriers or occlusive dressings can be used to increase skin permeability and enhance absorption.

Containers containing the composition of the invention can be used to facilitate the administration of glutamine according to the method of the invention. These containers are designed to contain, for example, the daily dosage of glutamine to be administered to the patient.

Containers adapted for intravenous administration of glutamine alone or in combination with other amino acids are especially useful. Such containers could comprise a receptacle for the liquid glutamine- containing composition, and a liquid conducive means capable of attachment to a needle.

The liquid conducive means could be any object capable of conveying the liquid composition in the receptacle to the needle such as, for example, plastic tubing.

The needle attached to the conducive means could either be inserted directly into a blood vessel of the human recipient or could be inserted into a reservoir to enable mixing with another solution before being administered to the patient.

The above disclosure generally describes the present invention. A more complete understanding can be obtained by reference to the following specific examples which are provided herein for purposes of illustration only and are not intended to be limiting unless otherwise specified.

### EXAMPLE 1

### The Effects of Glutamine Enriched Oral Diet on Small Bowel After Intestinal Resection

### Introduction

Compensatory growth of the small intestine after partial resection involves all coats of the bowel wall, but is dominated by villus hyperplasia. Increased villous height and crypt depth are accompanied by dilation and lengthening of the intestinal remnant, Williamson, R.C.N., "Intestinal adaption," N. Engl. J. Med., 298:1393-1444 (1978). Small bowel resection is followed by adaptive morphological and functional changes. Oral intake has been proved to be an important stimulus in the regulation of mucosal hyperplasia after intestinal resection, Levine, G.M., et al., "Small-bowel resection, oral intake is the stimulus for hyperplasia," Dig. Dis. 21:542-545 (1976). Luminal nutrients are important in maintaining normal mucosal growth and if oral intake is not maintained after resection of small bowel, the residual bowel loses its weight and becomes hypoplasic. Patients with short bowel after small bowel resection are supported by parenteral nutrition; their survival depends on the capacity of the residual intestinal system to adapt. The use of elemental diets and parenteral nutrition allows sufficient time for the development of intestinal adaptation and a slow return to complete oral intake, Weser, E., "The management of patients after small bowel resection," Gastroenterology 71:146-150 (1976).

Glutamine is an important fuel for enterocytes, and its utilization by the intestine appears to increase after surgical stress, Souba, W.W., et al., "Postoperative alteration of arteriovenous exchange of amino acids across the gastrointestinal tract," Surgery 94(2):342 (1983). Glutamine could serve as a local trophic factor, promoting mucosal growth when intestinal hypoplasia develops. This study investigated whether addition of glutamine to an elemental diet was associated with any advantage in terms of adaptation and recovery by the small intestine after subtotal resection. The study was designed to feed rats with a glutamine enriched elemental diet after a two-thirds small bowel resection. The mucosal adaptation of residual bowel was compared with controlled diet and sham-operated groups.

### Materials and Methods

Male wistar rats, weighing 175-200 gm, were purchased from the Charles River Breeding Laboratories, Inc., and allowed to acclimatize for 5 days. The rats were provided free access to water and fed with Purina chow diet. They were kept in individual cages and weighed every other day. After acclimatization, the rats with normal weight gain were randomly divided into four groups.

On the first day of study, the rats were anesthnetized with intraperitoneal injection of pentobarbital (50 mg/kg). The abdomen was opened via a midline incision, the whole length of small intestine from the ligament of Treitz to the ileocaecal valve was exteriorized and measured twice without stretch by a long black thread. The mean of the two measurements was determined. Then two-thirds resection of the small intestine beginning 5 cm distal to the ligament of Treitz was performed by the method of Lambert, R., "Surgery of the digestive system of the rats," Charles C. Thomas, Springfield, Illinois, pp32-35, 413-416 (1965). The resection margins were anastomosed end to end with 6-0 prolene. The intestine was replaced in the abdominal cavity and the abdominal wall was closed with 2-0 prolene. Control group was sham operated, undergoing transection and reanastomosis of the small intestine at the distal site, two-thirds of total length measured beginning 5 cm distal to the ligament of Treitz. The rats were kept in individual cages after operation and allowed to sip water on the first postoperative day.

Oral feeding was started from the second postoperative day. Rats in group 1 were orally alimented with glutamine enriched elemental diet (4.18%), and rats in group II were alimented with glycine enriched elemental diet (4.18%). Rats in group III (resected rats) and rats in group IV (sham-operated rats) were fed with ordinary chow diet. The feeding was continued for 7 days and their daily body weights were recorded until the day of harvest.

### Preparation of Glutamine and Glycine Elemental Diets

The elemental diet (NBC₀ Biochemicals, Inc., Cleveland, Ohio) contains 0.2% Choline chloride, 10% Corn oil, 46.9% Dextrin-white, 23.4% Surcose, 5% Salt mixture, 0.5% Vitamin mixture and 9.82% of 17 kinds essential and non-essential amino acids. Glutamine or glycine powder was added to the elemental diet to make 41.8% of glutamine or glycine enriched elemental diet, i.e. 28% of the total amino acids.

### Harvest Procedures

After 7 days of feeding, the rats were sacrificed. They were anesthetized with intraperitoneal injection of pentobarbital (50 mg/kg). The abdomen was opened and the incision extended to the chest cavity. Five ml of blood was drawn by puncture of the right ventricle for determination of blood ammonia and plasma glutamine levels. The intestine was harvested immediately following blood withdrawal. The entire small intestine was removed with careful separation of the mesentery, keeping close to the intestinal serosal. The removed intestine was suspended under fixed tension of 4.5 gm and 9 points were marked. For the rats with bowl resection, points of 5, 10 and 12.5 cm proximal to the anastmosis which represented the proximal jejunum and distal duodenum, and points of 2, 5 and 10 cm distal to the anastomosis which represented the residual proximal ileum close to the anastomosis, and points of 5, 10 and 15 cm proximal to the ileocaecal valve which represented the residual distal ileum, were marked with straight pins passed through the bowel. The six intestinal segments were separated. For the rats in sham-operated group, the proximal two segments were marked from 1 cm distal to the ligament of Treitz and measured upward. Segments 1a, 2a and 3a were rinsed in chilled saline and embedded in 10% buffered formalin for 4 hours, then transferred into 70% ethanol for fixation. Segments 1b, 2b and 3b were rinsed in chilled saline, their lumens were opened and were weighed wet weight. The intestinal segments were transferred into 5 ml of chilled saline and minced with sharp scissors. A homogenate was prepared, using two fifteen second periods on a Polytron homogenizer (Brinkman Instruments, Westbury, NY), followed by sonication with a sonicator (Heat system Laboratories, Plainview, NY) at a power setting of 2, for thirty seconds. Saline homogenates for DNA and protein analysis were stored at -30°C.

### Analytic Methods

The intestinal homogenates were analyzed for total protein according to the method of Lowry, O.H. et al., "Protein measurement with the Folin phenol reagent," J. Biochem., 193:265-275 (1951). DNA was determined according to the method of Burton, K., "A study of the conditions and mechanism of the diphenylamine reaction for the colorimetric estimation of deoxyribonucleic acid," Biochem, 62:315-323 (1965). Histologic sections were embedded in paraffin, stained with hematoxylin and eosin, and examined under a light microscope at 40X magnification. Twenty representative, tall, well-oriented complete villi were chosen to measure mucosal thickness and villous height using an eyepiece micrometer, and an average value obtained. The villus number was counted with the intestine put in the central horizontal line of 40X magnified field.

### Results

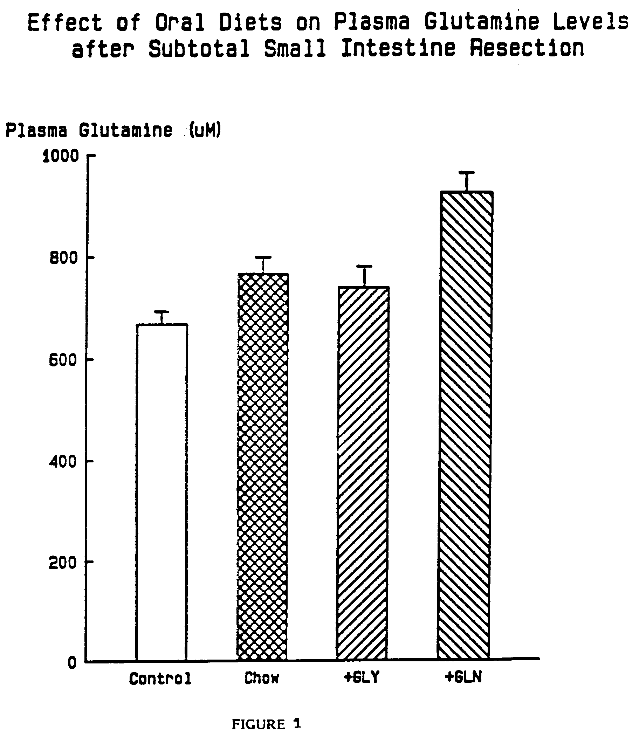

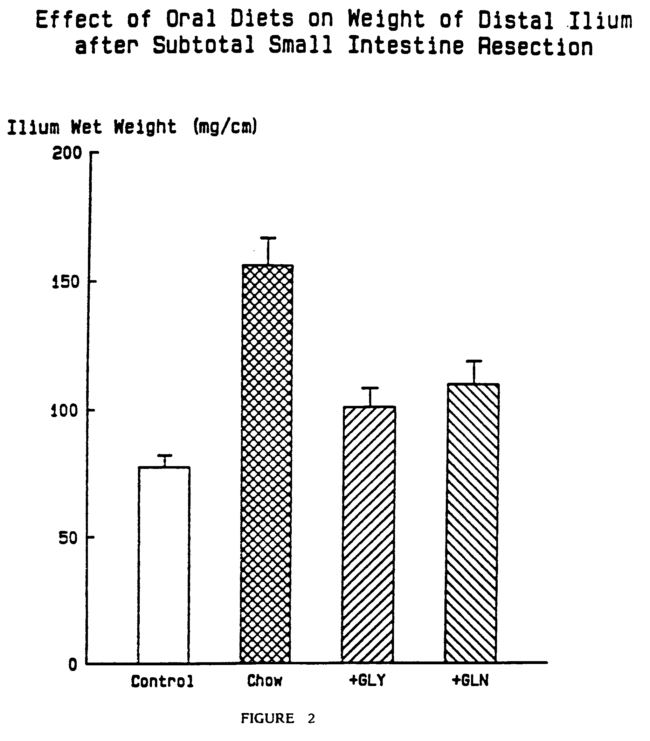

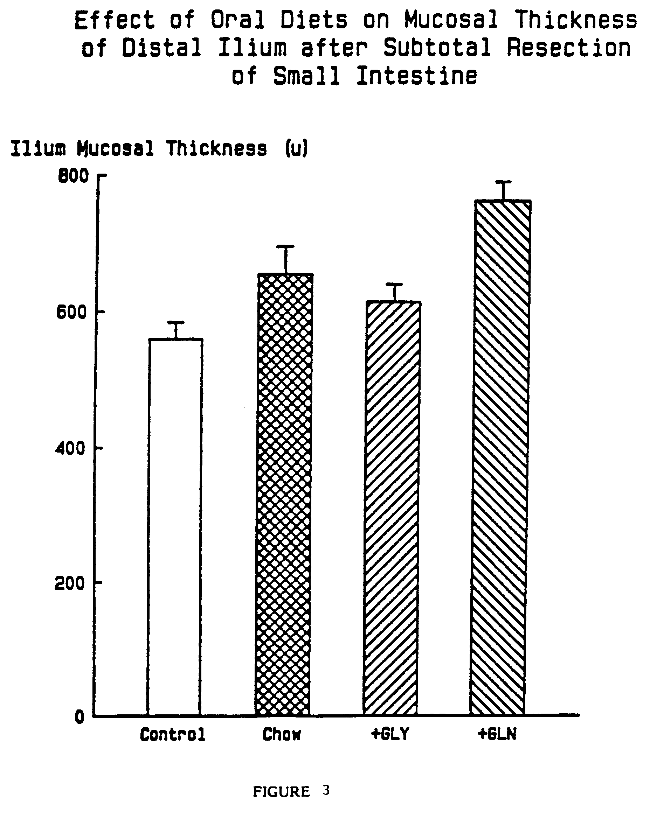

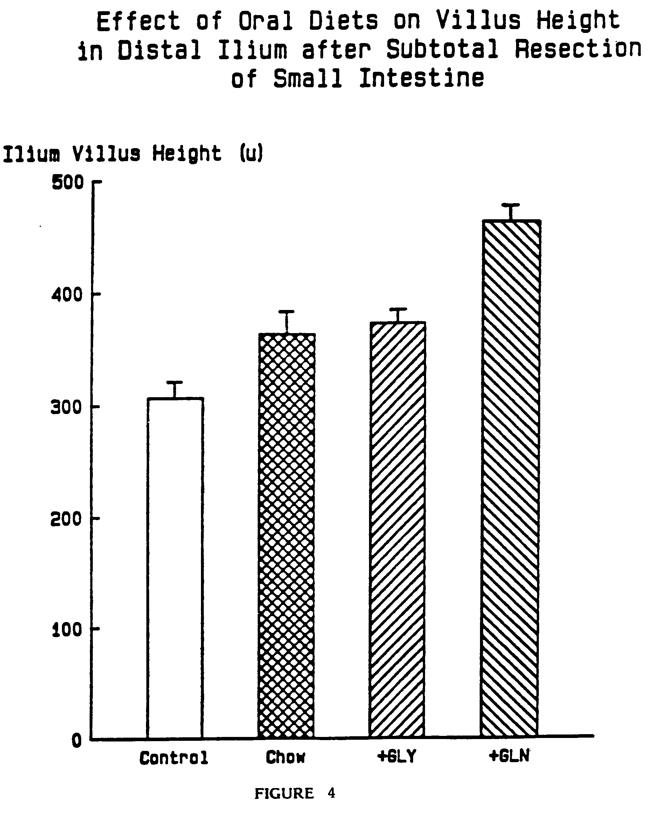

Figure 1 demonstrates that the glutamine-supplemented diet results in higher plasma glutamine levels. Based on measurements of weight/cm (Figure 2), there seemed to be little difference between the glutamine-supplemented (+GLN) and glutamine-free diets, with both supporting less intestinal hypertrophy than normal chow. However, on examination of intestinal mucosa with measurements of mucosal thickness (Figure 3 ) and villus height (Figure 4), the glutamine-supplemented diet proved superior.

### Example 2

### Preservation of Small Bowel Mucosa Using Glutamine-Enriched Parenteral Nutrition

Parenteral nutrition results in mucosal atrophy of the small intestine (Johnson, L.R. et al., Gastroenterology 68:1177-1183 (1975)). This response may be related to a decrease in gastrointestinal secretions and trophic hormones and also a relative lack of specific nutrients required for enterocyte proliferation. Glutamine is a major oxidative fuel for the small intestine but is not present in standard parenteral solutions. To determine the influence of dietary glutamine the small intestinal response to the administration of parenteral solutions enriched with varying concentrations of this amino acid was evaluated.

### MATERIALS AND METHODS

Conditioned male Wistar rats (n=42, wt 210-230g) underwent jugular venous catherterization and were fitted with a swivel assembly which allows long term infusion in unrestrained animals (Burt, M.E. et al., J. Physiol. 238:H599-603 (1980)). All rats were housed in individual metabolic cages and allowed access to drinking water. Control animals received 0.9% saline infusion and Purina rat chow ad libitum. Three groups of rats received intravenous nutrition. All nutrient solutions were isonitrogenous (0.9g nitrogen/100ml) and isocaloric (98Kcal/100ml), and contained equal concentrations of essential amino acids, nonessential amino acids and dextrose. The nonessential amino acid component of each solution was adjusted in order to provide glutamine concentrations of 0.2 or 3g/100ml. parenteral solutions were infused at a rate of 48ml/24hrs. Urine output and nitrogen excretion were measured daily. Animals were sacrificed following 7 days or parenteral nutrition and blood was obtained for determination of glutamine and ammonia concentrations. Both full thickness jejunal segments and mucosal samples were obtained from defned sections of the intestine. All samples were weighed, and homogenates were assayed for DNA and protein. Histologic paraffin sections of 5um thickness were prepared. Measurements of jejunal villus height, number and mucosal thickness were performed in a blinded fashion.

### RESULTS AND DISCUSSION

Wet weight, DNA, protein and villus height decreased in all rats receiving intravenous nutrition when compared to orally fed controls (Table I). Plasma glutamine concentration increased following infusion of solutions containing glutamine. Jejunal mucosal weight increased significantly when compared to rats receiving glutamine free solutions. Full thickness jejunal weight did tend to increase with glutamine intake although the response was not statistically significant. Both mucosal and full thickness jejunal DNA increased following glutamine infusion at 2 and 3% concentrations. These changes were accompanied by histological evidence of mucosal growth. Villus height and mucosal thickness increased in a dose dependent manner in proportion to quantity of glutamine administered. All animals were in positive nitrogen balance but rats receiving the 2% glutamine solution retained the greatest quantity of nitrogen throughout the study.

The provisions of glutamine in parenteral solutions results in an increase in jejunal mucosal weight, DNA content and villus height when animals are maintained on intravenous nutrition. An increase in the mucosal mass of the small intestine may improve small bowel function and facilitate the introduction of enteral nutrition. Glutamine may be a nutrient necessary for mucosal support which is not present in standard parenteral solutions at the present time.

**TABLE I**

| | **O GLN** | **2% GLN** | **3% GLN** | **CHOW** |
|---|---|---|---|---|
| | (n=10) | (n=11) | (n=10) | (n=11) |
| PLASMA GLN (umol/L) | 890.3±42.4 | 1105.8±98.8* | 717.1±46 | |
| FULL THICKNESS WT (mg/cm) | 30.6±0.8 | 31.4±1.0 | 32.±0.6 | 46.6±3.6 |
| MUCOSAL WT (mg/cm) | 17.7±0.7 | 20.4±1.1** | 20.3±1.0** | 29.3±2.3 |
| JEJUNAL DNA (ug/cm) | 252.8±9.5 | 279.9±7.3** | 303.1±19.4* | 370.2±33.0 |
| MUCOSAL DNA (ug/cm) | 101.7±5.9 | 134.0±7.1*** | 125.1±7.6** | 171.0±12.7 |
| VILLUS HT (um) | 266.5±8.4 | 294.0±7.9** | 306.4±9.9*** | 405.6±17.3 |
| MUCOSAL THICKNESS (um) | 422.6±9.7 | 448.1±8.9* | 452.6±11.5* | 589.6±20.9 |

| | | | | |
|---|---|---|---|---|
| *p<0.05, | | | | |
| **p<0.025, | | | | |
| ***p<0.005 vs O GLN | | | | |
| GLN = glutamine | | | | |

### EXAMPLE 3

### Effect of Glutamine - Enriched Parenteral Nutrition Following Treatment With 5-Fluorouricil

Addition of the amino acid glutamine (GLN) to nutrient solutions significantly enhances bowel cellularity during parenteral feeding. To evaluate the effect of GLN on mucosal regeneration following injury, 5 fluorouricil (5FU) was administered to rats receiving parenteral nutrition (PN). Following jugular venous catherization male Wistar rats (n=40, wt 200-230g) received a continuous infusion of isonitrogenous isocaloric solutions with (+) or without (-) GLN. 5FU was administered in increasing doses (0,100,150mg/kg i/p) to 24 animals at the initiation of PN (short-term) and to a further 16 animals (150mg/kg) who had received PN for 5 days prior to treatment (long-term). At harvest, 4 days following 5FU, WBC, Hb, platlets and plasma GLN were measured, jejunal samples were weighed, assayed for DNA and protein and histologic sections were prepared. A portion of the results are shown (mean ± SEM, *p <0.05, **p <0.025 compared to -GLN).

| | SHORT-TERM PARENTERAL NUTRITION | | | | LONG-TERM PN 150mg/kg 5FU | |
|---|---|---|---|---|---|---|
| | 0 mg/kg 5FU | | 150mg/kg 5FU | | | |
| | -GLN | +GLN | -GLN | +GLN | -GLN | +GLN |
| Survival | 100% | 100% | 85% | 85% | 25% | 75% |
| Mucosal Wt mg/cm | 22.6±1.1 | 28±1.8* | 15.6±1.4 | 22.7±3.1** | 10.7±1.0 | 15±1.0** |
| Mucosal DNA ug/cm | 151.7±7.4 | 188±13.5* | 79.9±6.7 | 113.7±19/7* | 59.2±8.3 | 72.7±5.2 |
| Muc Protein | 2.56±0.1 | 2.94±0.18 | 1.3±0.06 | 1.96±0.3** | 0.75±0.3 | 1.11±0.1 |

5FU treatment diminished intestinal weight, DNA and protein. Rats receiving GLN had significantly greater mucosal mass when compared to animals receiving standard solution. Addition of GLN was associated with increased survival in long-term animals. The provision of GLN in nutrient solutions improves small bowel cellularity and modifies the gastrointestinal toxicity of 5FU. Such therapy may diminish morbidity and mortality in patients whose gastrointestinal barrier defences are impaired.

## Claims

1. The use of glutamine or a functional analogue thereof that retains the characteristics of glutamine in the preparation of an agent for treating atrophy of small intestinal mucosa, the atrophy caused by catabolic dysfunction.

2. The use as claimed in claim 1 wherein administration of a therapeutically effective amount of the glutamine or analogue thereof is enteral or parenteral.

3. The use as claimed in claim 2 wherein the atrophy of intestinal mucosa that occurs is substantially associated with intravenous feeding.

4. The use as claimed in claim 1 or 2, wherein the atrophy occurs subsequent to physical trauma.

5. The use as claimed in claim 4 wherein the physical trauma is associated with surgery, sepsis, burn injuries, anorexia, chemotherapy, radiation therapy or uncontrolled diabetes.

6. The use as claimed in any of claims 1 to 5 wherein the administration is by use of a dietary supplement.

7. The use as claimed in any of claims 2 to 6 wherein the parenteral administration is by subcutaneous, intramuscular, or intravenous injection, nasopharyngeal or mucosal absorption, or transdermal absorption.

## Patentansprüche

1. Verwendung von Glutamin oder eines funktionellen Analogs davon, das die Merkmale von Glutamin beibehält, bei der Zubereitung eines Mittels zur Behandlung von Atrophie der Dünndarmschleimhaut, der durch katabolische Dysfunktion verursachten Atrophie.

2. Verwendung, wie beansprucht in Anspruch 1, wobei die Verabreichung einer therapeutisch wirksamen Menge des Glutamins oder eines Analogs davon enteral oder parenteral erfolgt.

3. Verwendung, wie beansprucht in Anspruch 2, wobei die auftretende Atrophie der Darmschleimhaut wesentlich mit intravenöser Ernährung in Zusammenhang steht.

4. Verwendung, wie beansprucht in Anspruch 1 oder 2, wobei die Atrophie im Anschluß an eine physikalische Verletzung auftritt.

5. Verwendung, wie beansprucht in Anspruch 4, wobei die physikalische Verletzung mit einem chirurgischen Eingriff, einer Sepsis, Verbrennungsverletzungen, Anorexie, Chemotherapie, Strahlentherapie oder unbehandeltem Diabetes zusammenhängt.

6. Verwendung, wie beansprucht in mindestens einem der Ansprüche 1 bis 5, wobei die Verabreichung durch Verwenden einer Nahrungsergänzung erfolgt.

7. Verwendung, wie beansprucht in mindestens einem der Ansprüche 2 bis 6, wobei die parenterale Verabreichung durch subkutane, intramuskuläre oder intravenöse Injektion, nasopharyngeale oder mucosale Absorption oder transdermale Absorption erfolgt.

## Revendications

1. Utilisation de glutamine ou d'un analogue fonctionnel de cette dernière qui conserve les caractéristiques de la glutamine dans la préparation d'un agent destiné au traitement de l'atrophie de la muqueuse de l'intestin grêle, l'atrophie étant provoquée par un dysfonctionnement catabolique.

2. Utilisation selon la revendication 1, où l'administration d'une quantité thérapeutiquement efficace de glutamine ou d'un analogue de cette dernière est entérale ou parentérale.

3. Utilisation selon la revendication 2, où l'atrophie de la muqueuse intestinale qui survient est sensiblement liée à l'alimentation intraveineuse.

4. Utilisation selon la revendication 1 ou 2, où l'atrophie survient à la suite d'un traumatisme physique.

5. Utilisation selon la revendication 4, où le traumatisme physique est lié à une intervention chirurgicale, une septicémie, des lésions par brûlure, une anorexie, une chimiothérapie, une radiothérapie ou un diabète non contrôlé.

6. Utilisation selon l'une quelconque des revendications 1 à 5, où l'administration s'effectue par recours à un supplément alimentaire.

7. Utilisation selon l'une quelconque des revendications 2 à 6, où l'administration parentérale se fait par injection sous-cutanée, intramusculaire ou intraveineuse, par absorption nasopharingienne ou muqueuse ou par absorption transdermique.
